(19) Europäisches Patentamt
European Patent Office
Office européen des brevets

(11) **EP 3 653 605 B1**

(12) **EUROPÄISCHE PATENTSCHRIFT**

(45) Veröffentlichungstag und Bekanntmachung des Hinweises auf die Patenterteilung:
**13.10.2021   Patentblatt 2021/41**

(51) Int Cl.:
***C07C 263/10*** *(2006.01)*       ***C07C 265/14*** *(2006.01)*

(21) Anmeldenummer: **19208661.9**

(22) Anmeldetag: **12.11.2019**

(54) **VERFAHREN ZUR HERSTELLUNG EINES ISOCYANATS DURCH TEILWEISE ADIABATISCH BETRIEBENE PHOSGENIERUNG DES KORRESPONDIERENDEN AMINS**

METHOD FOR THE PREPARATION OF ISOCYANATE BY PARTIALLY ADIABATIC PHOSGENATION OF THE CORRESPONDING AMINE

PROCÉDÉ DE PRODUCTION D'UN ISOCYANATE PAR PHOSGÉNATION À FONCTIONNEMENT PARTIELLEMENT ADIABATIQUE D'AMINE CORRESPONDANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.11.2018   EP 18205954**

(43) Veröffentlichungstag der Anmeldung:
**20.05.2020   Patentblatt 2020/21**

(73) Patentinhaber: **Covestro Intellectual Property GmbH & Co. KG
51373 Leverkusen (DE)**

(72) Erfinder:
• **KNAUF, Thomas
  41542 Dormagen (DE)**
• **STEFFENS, Christian
  25436 Tornesch (DE)**
• **HIELSCHER, Anke
  51063 Köln (DE)**
• **WASTIAN, Dietmar
  41542 Dormagen (DE)**
• **SPRIEWALD, Jürgen
  Seabrook, TX 77586 (US)**

(74) Vertreter: **Levpat
c/o Covestro AG
Gebäude 4825
51365 Leverkusen (DE)**

(56) Entgegenhaltungen:
**EP-A1- 1 616 857**

EP 3 653 605 B1

**Beschreibung**

Die vorliegende Erfindung betrifft ein Verfahren zur Herstellung eines Isocyanats durch Umsetzung eines primären Amins mit Phosgen umfassend **I**) Bereitstellung einer Aminlösung und Temperierung derselben in einem Wärmeaustauscher, **II**) Bereitstellung einer Phosgenlösung und Temperierung derselben in einem Wärmeaustauscher, **III**) Vermischen der Amin- mit der Phosgenlösung in einer Mischeinrichtung gefolgt von **IV**) weiterer Umsetzung in einer adiabatisch betriebenen Reaktionszone und dem Abtrennen der infolge der chemischen Umsetzung gebildeten Gasphase in einer Trennzone, **V**) Entspannen der verbleibenden Flüssigphase, **VI**) weitere Umsetzung der nach Entspannung verbleibenden Flüssigphase in einer indirekt beheizten Reaktionszone und **VII**) Isolierung des Isocyanats aus der dort erhaltenen Reaktionslösung, bei welchem man die Temperatur in der Reaktionszone und Trennzone einstellt, indem man für die Temperatur des Reaktionsgemisches aus Schritt III) einen Soll-Wert innerhalb eines Bereiches von 110 °C bis 145 °C festlegt und die kontinuierlich oder in Intervallen gemessene Ist-Temperatur des Reaktionsgemisches aus Schritt III) zur Regelung der Temperatur der in Schritt I) bereitgestellten Lösung des primären Amins und/oder der Temperatur der in Schritt II) bereitgestellten Lösung von Phosgen mittels der zur Temperierung dieser Lösungen jeweils eingesetzten Wärmeaustauscher verwendet.

Isocyanate (1) werden in großen Mengen hergestellt und dienen hauptsächlich als Ausgangsstoffe zur Herstellung von Polyurethanen. Ihre Herstellung erfolgt zumeist durch Umsetzung der entsprechenden Amine (2) mit Phosgen (3), wobei Phosgen im stöchiometrischen Überschuss eingesetzt wird. Die Umsetzung der Amine mit dem Phosgen kann sowohl in der Gasphase als auch in der Flüssigphase erfolgen, wobei die Reaktion diskontinuierlich oder kontinuierlich durchgeführt werden kann. Die Phosgenierungsreaktion liefert - im Fall der Gasphasenphosgenierung nach der sog. *Quenche* des zunächst anfallenden gasförmigen Reaktionsprodukts - eine Flüssigphase enthaltend das gewünschte Isocyanat. Neben dieser Flüssigphase fallen an verschiedenen Stellen des Prozesses Gasströme an, die, nachdem diese soweit technisch möglich und wirtschaftlich sinnvoll von Wertprodukten wie Chlorwasserstoff, Phosgen, Isocyanat und Lösungsmittel befreit wurden, im Allgemeinen einer Phosgenzersetzung zugeführt werden, in welcher Spurenanteile an Phosgen, die in den vorangegangenen Aufarbeitungsschritten nicht abgetrennt werden konnten, katalytisch mit Wasser zersetzt werden. Im Allgemeinen wird hierfür Aktivkohle als Katalysator eingesetzt. Diese Phosgenzersetzung liefert ein gereinigtes Abgas und einen sauren Abwasserstrom, der entsorgt werden muss. Dieser Abwasserstrom enthält im Allgemeinen noch organische Verunreinigungen wie beispielsweise Lösungsmittel (im Fall der Herstellung von MDI üblicherweise Monochlorbenzol), Amin (im Fall der Herstellung von MDI Anilin) und Harnstoffverbindungen. Diese organischen Verunreinigungen müssen weitestgehend entfernt werden, bevor das Abwasser einer Abwasseraufbereitungsanlage (zum Beispiel einer biologischen Kläranlage) zugeführt werden kann. Eine Möglichkeit, dies zu erreichen, besteht darin, dass Abwasser durch Zugabe von Base (zum Beispiele Natronlauge) auf einen pH-Wert > 7, insbesondere im Bereich von 11 bis 13, zu bringen und im Anschluss die organischen Verunreinigungen an Aktivkohle zu adsorbieren. Eine solche Adsorption an Aktivkohle ermöglicht auf einfache Weise die Reduktion der Konzentration an organischen Verunreinigungen in diesem Abwasserstrom auf ein Niveau, das es erlaubt, das Abwasser einer Abwasseraufbereitungsanlage zuzuführen.

Verfahren zur Herstellung von organischen Isocyanaten aus primären Aminen und Phosgen sind bereits vielfach beschrieben worden; rein exemplarisch sei auf die folgenden Schriften verwiesen:
DE-A-34 03 204 beschreibt ein kontinuierliches Verfahren zur Herstellung organischer Polyisoyanate, bei welchem bei einem Druck von 5 bis100 bar in einer zum Teil im Kreislauf geführten Reaktion erhöhte Temperaturen von 100 bis 220 °C eingestellt werden.

DE-A-17 68 439 beschreibt ein Verfahren zur fortlaufenden Herstellung organischer Isocyanate, bei welchem die Einsatzstoffe Amin und Phosgen zunächst vorerhitzt und dann die vorerhitzten Bestandteile in der Reaktionszone unter hohen Drücken zusammengeführt und unter isothermen Bedingungen, d. h. unter Wärmeaustausch mit der Umgebung, zur Reaktion gebracht werden.

DE-A-102 22 968 beschreibt ein Verfahren zur kontinuierlichen Herstellung von Polyisocyanaten durch Umsetzung von primären Aminen mit Phosgen, bei welchem die Umsetzung in einer Kaskade von temperierbaren Reaktionsrohren unterschiedlicher Größe durchgeführt wird.

EP 1 873 142 A1 beschreibt eine dreistufige Verfahrensführung, bei welcher der Druck zwischen der ersten Stufe eines Mischers und der zweiten Stufe eines ersten Phosgenierreaktors gleichbleibt oder ansteigt und in der dritten Stufe, einem Apparat zur Phosgenabtrennung, der Druck niedriger ist als in der zweiten Stufe. Die Reaktion kann adiabatisch oder isotherm betrieben werden.

Von großtechnischem Interesse sind sowohl aromatische Isocyanate wie beispielsweise Methylen-Diphenylen-Diisocyanat (fortan MMDI - "monomeres MDI"), Gemische aus MMDI und Polymethylen-Polyphenylen-Polyisocyanate (das sind die höheren Homologen des MMDI, fortan PMDI, "polymeres MDI") oder Toluylendiisocyanat (TDI) als auch aliphatische Isocyanate wie z. B. 1,5-Pentandiisocyanat (PDI), 1,6-Hexamethylendiisocyanat (HDI), oder Isophorondiisocyanat (IPDI). Daneben sind auch Isocyanate mit benzylischen Isocyanatgruppen bedeutsam, insbesondere sei hier Xylylendiisocyanat (XDI) erwähnt. Die vorliegende Erfindung befasst sich insbesondere mit der Herstellung von Methylen-Diphenylen-Diisocyanaten und Polymethylen-Polyphenylen-Polyisocyanaten (fortan summarisch MDI genannt).

Bei der Mehrzahl der bekannten Verfahren werden zur Einstellung der gewünschten Reaktionstemperatur temperierbare Reaktoren in unterschiedlichen Varianten (Mantelheizung, Beheizung durch Wärmeaustauscher oder spezielle Reaktoreinbauten) eingesetzt. Bei der Isocyanatsynthese durch Phosgenierung von Aminen stellt die externe Temperierung der Reaktoren jedoch oft ein Problem dar, da die hohen Temperaturen der Reaktorwandflächen die Bildung von Nebenprodukten fördern oder sogar erst verursachen, welche dann die Ausbeute und/oder Produkteigenschaften nachteilig beeinflussen. Außerdem werden dann im Reaktor Ablagerungen gebildet, die ein regelmäßiges Abfahren und Reinigen der Reaktoren erforderlich machen. Dies führt aber zum Verlust von Anlagenkapazität und somit zu einem wirtschaftlichen Nachteil. Darüber hinaus verursachen die Wärmeträgeranlagen zusätzliche Investitionskosten, was die Wirtschaftlichkeit des Verfahrens ebenfalls verschlechtert. Zur Lösung dieser Probleme wird in EP 1616 857 A1 eine zweistufige Verfahrensführung vorgeschlagen, bei welcher in einer ersten Stufe a) Amin und Phosgen in einer *adiabatisch geführten Reaktion* zur Umsetzung gebracht werden, wobei die Temperatur der Umsetzung auf Werte zwischen 100 und 220 °C begrenzt wird, indem der absolute Druck im Reaktor durch Entspannung gezielt auf Werte zwischen 8 und 50 bar eingestellt wird, und die Temperatur bei Werten zwischen 100 und 220 °C so lange beibehalten wird, bis ein Umsatz an Phosgen von mindestens 80 % erreicht ist, und anschließend in einer zweiten Stufe b) das Reaktionsgemisch aus der ersten Stufe auf absolute Drücke im Bereich von 1 bis 15 bar entspannt und bei Temperaturen zwischen 90 und 240°C, *üblicherweise unter Zufuhr von Wärme,* weiter umgesetzt wird. Eine solche Verfahrensführung kann als *adiabatisch-isotherme Verfahrensführung* bezeichnet werden. Wesentlich für das beschriebene Verfahren ist die Einstellung der Temperatur der Umsetzung im adiabatisch betriebenen Reaktor (100 °C bis 220 °C, bevorzugt 115 °C bis 180 °C, besonders bevorzugt 120 °C bis 150 °C) *durch den Druck* in diesem Reaktor. Diese Einstellung durch den Druck erfolgt durch kontrolliertes Entspannen (vgl. Absatz [0016]). Eine *Einstellung* der Temperatur im adiabatisch betriebenen Reaktor durch *gezielte Regelung von* Edukt-Temperaturen wird in EP 1 616 857 A1 nicht offenbart. Das den adiabatisch betriebenen Reaktor verlassende Reaktionsgemisch wird in einer zweiten Stufe unter isothermen Bedingungen weiter umgesetzt und dabei auf einen Druck unterhalb desjenigen der ersten Stufe entspannt (vgl. Absatz [0019]). Am Ausgang des isotherm betriebenen Reaktors werden diesem eine Gasphase und eine das Isocyanat enthaltende Flüssigphase separat entnommen.

Die Güte eines Verfahrens zur Herstellung von Isocyanaten ist einerseits definiert durch den Gehalt an unerwünschten Nebenprodukten im Produkt des Verfahrens. Andererseits ist die Güte eines Verfahrens dadurch definiert, dass der gesamte Prozess von Anfahren, Produktion im Regelbetrieb bis zum Abfahren des Prozesses ohne technischen Produktionsausfall und ohne Probleme, die zu einem Eingriff in den Prozess nötigen würden, betrieben werden kann, und dass es nicht zu Verlusten an Einsatzstoffen, Zwischenprodukten oder an Endprodukt kommt.

Idealerweise sind daher die großtechnischen Anlagen zur Durchführung solcher Herstellungsverfahren derart ausgelegt, dass bei einer passenden Qualität der eingesetzten Hilfs-und Einsatzstoffe und richtiger Wahl der Verfahrensparameter wie Druck, Temperatur, Mengenverhältnisse und Konzentrationen der Hilfs- und Einsatzstoffe etc. die Verfahren robust verlaufen. Das bedeutet, dass es in solchen kontinuierlich betriebenen Großanlagen idealerweise nicht zu Problemen wie etwa der Entstehung von Niederschlägen kommt, die sich an Anlagenteilen absetzen und beispielsweise Rohrleitungen verstopfen können. Andererseits trägt auch eine optimale Ausnutzung der Raum-Zeit-Ausbeute in einem nicht unerheblichen Umfang zu einer Verbesserung der Produktivität und somit der Wirtschaftlichkeit von großtechnischen Phosgenieranlagen bei. Geht man bei der Produktionsrate in die Grenzbereiche des noch Machbaren einer gegebenen Anlage, dann müssen die Verfahrensparameter in einem sehr engen Fenster betrieben werden, damit es nicht zu den oben geschilderten Problemen mit Niederschlägen, Anbackungen und Produktqualität kommt.

Zusammenfassend kann daher festgestellt werden, dass die zum Betrieb einer Phosgenieranlage erforderlichen Steuerungs- und Regelungsprozesse daraufhin optimiert sein sollten, eine energieeffiziente Phosgenierung mit minimalem apparativen Aufwand unter weitestgehender Vermeidung der Bildung von Ablagerungen und Nebenprodukten insbesondere im Reaktor und unter gleichzeitiger Erzielung der angestrebten Produktqualität zu bewirken. Eine solche Optimierung des Phosgenierverfahrens wirkt sich auch positiv auf den Bedarf an Lösungsmittel und den erforderlichen Phosgenüberschuss aus. In dieser Hinsicht bestand noch Verbesserungsbedarf gegenüber dem Stand der Technik.

Diesem Bedarf Rechnung tragend ist ein Gegenstand der vorliegenden Erfindung ein Verfahren **zur Herstellung eines Isocyanats durch Umsetzung eines primären Amins mit Phosgen,** umfassend die Schritte:

I) Bereitstellen einer Lösung des primären Amins in einem Lösungsmittel und Temperieren der Lösung des primären Amins durch indirekte Wärmeübertragung in einem mit einem Wärmeträgermedium betriebenen Wärmeaustauscher;

II) Bereitstellen einer Lösung von Phosgen in einem Lösungsmittel und Temperieren der Lösung von Phosgen durch indirekte Wärmeübertragung in einem mit einem Wärmeträgermedium betriebenen Wärmeaustauscher;

III) Vermischen der in Schritt I) bereitgestellten Lösung des primären Amins und der in Schritt II) bereitgestellten Lösung von Phosgen in einer Mischeinrichtung zu einem Reaktionsgemisch unter Einhaltung eines auf die Aminogruppen des primären Amins bezogenen stöchiometrischen Überschusses an Phosgen im Bereich von 40 % bis 200 % der Theorie, bevorzugt im Bereich von 40 % bis 120 % der Theorie, besonders bevorzugt im Bereich von 50 % bis 100 % der Theorie, ganz besonders bevorzugt im Bereich von 50 % bis 75 % der Theorie;

IV) Führen des in Schritt III) erhaltenen flüssigen Reaktionsgemisches durch eine Reaktionszone und durch eine dieser Reaktionszone strömungstechnisch nachgeschaltete Trennzone unter Ausbildung einer unter einem Druck im Bereich von 8,0 bar$_{(abs.)}$ bis 50,0 bar$_{(abs.)}$, insbesondere im Bereich von 15,0 bar$_{(abs.)}$ bis 30,0 bar$_{(abs.)}$, stehenden Gasphase aus dem flüssigen Reaktionsgemisch in der Trennzone, wobei die Reaktionszone und die Trennzone nicht beheizt und nicht gekühlt werden, wobei die in der Trennzone gebildete Gasphase und die verbleibende Flüssigphase der Trennzone getrennt voneinander entnommen werden;

wobei man die Temperatur in der Reaktionszone und Trennzone einstellt, indem man für die Temperatur des Reaktionsgemisches aus Schritt III) einen Soll-Wert innerhalb eines Bereiches von 110 °C bis 145 °C festlegt und die kontinuierlich oder in Intervallen gemessene Ist-Temperatur des Reaktionsgemisches aus Schritt III) zur Regelung

der Temperatur der in Schritt I) bereitgestellten Lösung des primären Amins auf einen Wert im Bereich von 30 °C bis 130 °C, insbesondere im Bereich von 60 °C bis 100 °C, mittels des in Schritt I) eingesetzten Wärmeaustauschers

und/oder (bevorzugt *und*)

der Temperatur der in Schritt II) bereitgestellten Lösung von Phosgen auf einen Wert im Bereich von -20 °C bis 120 °C, insbesondere im Bereich von -10 °C bis 30 °C, mittels des in Schritt II) eingesetzten Wärmeaustauschers verwendet;

V) Entspannen der der Trennzone aus Schritt IV) entnommenen Flüssigphase unter partieller Überführung dieser Flüssigphase in die Gasphase;

VI) Führen der in Schritt V) nach dem Entspannen verbleibenden Flüssigphase durch eine indirekt beheizte Reaktionszone, wobei sich eine Chlorwasserstoff- und Phosgen-haltige Gasphase (die im Allgemeinen auch noch Anteile verdampften Lösungsmittels enthält) ausbildet, die abgetrennt wird, und eine Isocyanat- und Lösungsmittel enthaltende Flüssigphase verbleibt, die der indirekt beheizten Reaktionszone entnommen wird;

VII) Aufarbeiten der in Schritt VI) gewonnenen Isocyanat- und Lösungsmittel enthaltenden Flüssigphase unter Rückgewinnung des Lösungsmittels und Erhalt des Isocyanats.

Erfindungsgemäß steht die sich in der Trennzone ausbildende Gasphase *"unter einem Druck im Bereich von 8,0 bar$_{(abs.)}$ bis 50,0 bar$_{(abs.)}$, insbesondere im Bereich von 15,0 bar$_{(abs.)}$ bis 30,0 bar$_{(abs.)}$ "*. Durch die in der Reaktionszone ablaufenden chemischen Umsetzungen bildet sich aus dem in Schritt III) erhaltenen flüssigen Reaktionsgemisch eine (mindestens) Chlorwasserstoff-und Phosgen-haltige Gasphase. Die genannten Druckwerte beziehen sich also auf den Gasraum der Trennzone. Hier und im Folgenden sind alle Drücke als absolute Drücke (gekennzeichnet als "bar$_{(abs)}$") zu verstehen.

Da die Trennzone der Reaktionszone *"strömungstechnisch nachgeschaltet"* ist, womit in der Terminologie der vorliegenden Erfindung auch eine offene, strömungstechnische Verbindung beider Zonen gemeint ist, und da ferner die Reaktions- und Trennzone *"nicht beheizt und nicht gekühlt werden"* (= adiabatische Verfahrensführung), stellt sich an jeder Stelle der Reaktions- und Trennzone eine Temperatur ein, die bei gegebener Temperatur des Reaktionsgemisches aus Schritt III) im Wesentlichen - abgesehen von Wärmeverlusten durch nicht perfekte Isolierung der verwendeten Apparaturen - von den Reaktionsenthalpien der ablaufenden chemischen Prozesse (die weiter unten noch im Detail erläutert werden) bestimmt wird. Auch der sich etablierende Druck wird zunächst von den ablaufenden chemischen Prozessen bestimmt. Vorzugsweise ist jedoch in der Trennzone ein Druckhalteventil für die sich ausbildende Gasphase und eine Flüssigkeitsstandregelung für die Flüssigphase vorgesehen, um sicher gewährleisten zu können, dass Druck im oben genannten Bereich - 8,0 bar$_{(abs.)}$ bis 50,0 bar$_{(abs.)}$, insbesondere im Bereich von 15,0 bar$_{(abs.)}$ bis 30,0 bar$_{(abs.)}$ - liegt und, einmal auf einen gewünschten Wert innerhalb dieses Bereiches eingestellt, während des Betriebs - gegebenenfalls mit Ausnahme von nicht stationären Zuständen wie Lastwechseln oder Betriebsstörungen - *konstant bleibt.* Somit hängt aber die Temperatur in Schritt IV) letztlich von der Temperatur *des diesem Schritt zuzuführenden Reaktionsgemisches* ab. Der Ist-Wert dieser Temperatur wird erfindungsgemäß kontinuierlich oder in Intervallen gemessen und mit einem zuvor festgelegten Soll-Wert innerhalb des erfindungsgemäß geforderten Bereiches von 110 °C bis 145 °C verglichen. Weicht der gemessene Ist-Wert vom Soll-Wert signifikant ab (was insbesondere eine Abweichung von mehr als 1,0 °C, bevorzugt von mehr als 0,5 °C bedeutet), so wird die Temperatur der in Schritt I) bereitgestellten Lösung des primären Amins und/oder die Temperatur der in Schritt II) bereitgestellten Lösung von Phosgen entsprechend angepasst (also bei einer Abweichung nach unten erhöht und bei einer Abweichung nach oben erniedrigt), um den Ist-Wert dem Soll-Wert anzugleichen. Dies wird im Rahmen der erfindungsgemäßen Terminologie als Verwendung der gemessenen Ist-Temperatur des Reaktionsgemisches aus Schritt III) *"zur Regelung der Temperatur der in Schritt I) bereitgestellten Lösung des primären Amins ... und/oder der Temperatur der in Schritt II) bereitgestellten Lösung von Phosgen ... "* bezeichnet. Dabei ist es bevorzugt, beide Temperaturen, also die Temperatur der in Schritt I) bereitgestellten Lösung des primären Amins und die Temperatur der in Schritt II) bereitgestellten Lösung von Phosgen, entsprechend anzupassen. Dies kann zeitgleich (parallel) oder zeitversetzt geschehen.

Der in der Terminologie der Erfindung verwendete Begriff "*Wärmeträgermedium"* umfasst also auch den Fall, dass mittels des Wärmeaustauschers eine Abkühlung herbeigeführt wird (und man in diesem Fall daher auch von einem" *Kühlmedium* " sprechen könnte).

Aufgrund der adiabatischen Bedingungen in der Reaktions- und Trennzone aus Schritt IV) besteht somit ein reproduzierbarer Zusammenhang zwischen der Temperatur der der Reaktionszone aus Schritt IV) zugeführten Reaktionsmischung aus Schritt III) und der Temperatur in der Reaktions- und Trennzone, die vorteilhafterweise als Temperatur der die Trennzone verlassenden Flüssigphase bestimmt werden kann.

Erfindungsgemäß wird Phosgen, bezogen auf die Aminogruppen des primären Amins, im *"stöchiometrischen Überschuss"* eingesetzt. Theoretisch reagiert 1 Mol Phosgen mit 1 Mol primärer Aminogruppen (1 R-NH$_2$ + 1 COCl$_2$ → 1 R-NCO + 2 HCl). Ein Phosgenüberschuss von x % gegenüber primären Aminogruppen entspricht daher einem molaren Verhältnis $_n$(Phosgen)/$_n$(-NH$_2$) (n = Stoffmenge) von $\dfrac{1+\frac{x}{100}}{1}$, also beispielsweise $\dfrac{1+\frac{40}{100}}{1}=1{,}40$ bei 40%igem Phosgenüberschuss oder beispielsweise $\dfrac{1+\frac{120}{100}}{1}=2{,}2$ bei 120%igem Phosgenüberschuss.

Es folgt zunächst eine Kurzzusammenfassung verschiedener möglicher **Ausführungsformen der Erfindung:** In einer **ersten Ausführungsform** der Erfindung, die mit allen anderen Ausführungsformen kombiniert werden kann, weist die in Schritt I) bereitgestellte Lösung des primären Amins einen auf die Gesamtmasse dieser Lösung bezogenen Massenanteil an primärem Amin im Bereich von 25 % bis 50 %, insbesondere im Bereich von 30 % bis 45 %, auf, und die in Schritt II) bereitgestellte Lösung von Phosgen weist einen auf die Gesamtmasse dieser Lösung bezogenen Massenanteil an Phosgen im Bereich von 45 % bis 90 %, insbesondere im Bereich von 55 % bis 80 %, auf.

In einer **zweiten Ausführungsform** der Erfindung, die mit allen anderen Ausführungsformen kombiniert werden kann, werden die in den Schritten IV), V) und VI) anfallenden Gasphasen zur Gewinnung von Chlorwasserstoff und Phosgen sowie gegebenenfalls Lösungsmittel aufgearbeitet.

In einer **dritten Ausführungsform** der Erfindung, die eine besondere Ausgestaltung der zweiten Ausführungsform ist, werden die in den Schritten IV), V) und VI) anfallenden Gasphasen vor der Aufarbeitung auf einen gemeinsamen Druck eingestellt und vereinigt.

In einer **vierten Ausführungsform** der Erfindung, die mit allen anderen Ausführungsformen kombiniert werden kann, wird die Mischeinrichtung aus Schritt III) nicht beheizt und nicht gekühlt.

In einer **fünften Ausführungsform** der Erfindung, die mit allen anderen Ausführungsformen kombiniert werden kann, umfasst in Schritt III) eingesetzte Mischeinrichtung einen oder mehrere dynamischen Mischer und umfasst insbesondere keinen statischen Mischer.

In einer **sechsten Ausführungsform** der Erfindung, die mit allen anderen Ausführungsformen kombiniert werden kann, werden die Reaktionszone und Trennzone aus Schritt IV) in einem gemeinsamen Reaktor angeordnet.

In einer **siebten Ausführungsform** der Erfindung, die eine besondere Ausgestaltung der sechsten Ausführungsform ist, wird als Reaktor ein aufrecht angeordneter röhrenförmiger Reaktor (Rohrreaktor, Turmreaktor) eingesetzt.

In einer **achten Ausführungsform** der Erfindung, die eine besondere Ausgestaltung der siebten Ausführungsform ist, wird der Reaktor mit dem in Schritt III) erhaltenen Reaktionsgemisch von unten nach oben durchströmt.

In einer **neunten Ausführungsform** der Erfindung, die mit allen anderen Ausführungsformen kombiniert werden kann, ist die indirekt beheizte Reaktionszone aus Schritt VI) Bestandteil eines Rohrbündelreaktors (Reaktor aufweisend eine Mehrzahl an Reaktionsrohren angeordnet in einen diese umhüllenden Reaktorbehälter, sodass sich ein Rohrinnenraum - das Innere der Reaktionsrohre - und ein Rohraußenraum - der Raum zwischen den Reaktionsrohren, nach außen begrenzt durch die Innenwand des umhüllenden Reaktorbehälters - ausbilden), durch dessen Rohrinnenraum die in Schritt V) nach dem Entspannen verbleibende Flüssigphase geführt wird und durch dessen Rohraußenraum ein Heizmedium geführt wird, oder durch dessen Rohraußenraum die in Schritt V) nach dem Entspannen verbleibende Flüssigphase geführt wird und durch dessen Rohrinnenraum ein Heizmedium geführt wird.

In einer **zehnten Ausführungsform** der Erfindung, die mit allen anderen Ausführungsformen kombiniert werden kann, werden die in Schritt I) und in Schritt II) eingesetzten Wärmeaustauscher unabhängig voneinander ausgewählt aus der Gruppe bestehend aus Rohrbündelwärmeaustauschern und Plattenwärmeaustauschern, wobei es bevorzugt ist, in beiden Schritten den gleichen Typ von Wärmemaustauscher einzusetzen.

In einer **elften Ausführungsform** der Erfindung, die mit allen anderen Ausführungsformen kombiniert werden kann, werden die in Schritt I) und in Schritt II) eingesetzten Wärmeaustauscher unabhängig voneinander mit einem Wärmeträgermedium ausgewählt aus der Gruppe bestehend aus Öl, Salzschmelzen, organischen Lösungsmitteln und Wasser betrieben werden, wobei organische Lösungsmittel (bevorzugt Monochlorbenzol, Dichlorbenzol oder Toluol, besonders bevorzugt Monochlorbenzol) bevorzugt sind.

In einer **zwölften Ausführungsform** der Erfindung, die mit allen anderen Ausführungsformen kombiniert werden kann, sind in der Trennzone aus Schritt IV) ein Druckhalteventil für die sich ausbildende Gasphase und eine Flüssigkeitsstandregelung für die Flüssigphase angeordnet, durch welche der Druck der Gasphase konstant gehalten wird.

In einer **dreizehnten Ausführungsform** der Erfindung, die mit allen anderen Ausführungsformen kombiniert werden kann, umfasst Schritt IV) Folgendes:

- Eintragen des Soll-Werts für die Temperatur des Reaktionsgemisches aus Schritt III) in eine elektronische Datenverarbeitungsanlage;
- Bereitstellen der Messwerte der Ist-Temperatur des Reaktionsgemisches aus Schritt III) in elektronischer Form;
- Übermitteln der in elektronischer Form bereitgestellten Messwerte an die elektronische Datenverarbeitungsanlage;
- Abgleichen des Soll-Werts mit der Ist-Temperatur in der elektronischen Datenverarbeitungsanlage;
- bei festgestellter Abweichung der Ist-Temperatur vom Soll-Wert um mehr als 1,0 °C (bevorzugt um mehr als 0,5 °C):

  ○ bei Abweichung nach oben, Reduzieren
  ○ bei Abweichung nach unten, Erhöhen

der Temperatur des in den Wärmeaustauschern verwendeten Wärmeträgermediums durch Übermitteln eines entsprechenden Befehls aus der elektronischen Datenverarbeitungsanlage an die zur Temperierung des Wärmeträgermediums eingesetzte Temperier-Vorrichtung.

In einer **vierzehnten Ausführungsform** der Erfindung, die mit allen anderen Ausführungsformen kombiniert werden kann, wird

(i) Methylen-Diphenylen-Diisocyanat und/oder Polymethylen-Polyphenylen-Polyisocyanat durch Umsetzung von Methylen-Diphenylen-Diamin und/oder Polymethylen-Polyphenylen-Polyamin mit Phosgen oder

(ii) Toluylendiisocyanat durch Umsetzung von Toluylendiamin mit Phosgen

hergestellt.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

**Schritt I**) der vorliegenden Erfindung, die Bereitstellung der für die Phosgenierung erforderlichen Aminlösung, kann nach allen aus dem Stand der Technik bekannten Verfahren erfolgen. Das einzusetzende Amin wird durch das angestrebte Isocyanat bestimmt. Das erfindungsgemäße Verfahren ist grundsätzlich zur Herstellung beliebiger aromatischer, aliphatischer und araliphatischer Isocyanate geeignet. Bevorzugt eingesetzt wird das erfindungsgemäße Verfahren zur Herstellung von Methylen-Ddiphenylen-Diisocynat (aus Methylen-Diphenylen-Diamin), Polymethylen-Polyphenylen-Polyisocyanat (aus Polymethylen-Polyphenylen-Polyamin), Gemischen aus Methylen-Diphenylen-Diisocyanat und Polymethylen-Polyphenylen-Polyisocyanat (diese Gemische werden fortan auch als MDI und die Ausgangsamin-Gemische als MDA bezeichnet), Toluylendiisocyanat (aus Toluylendiamin), Xylylendiisocyanat (aus Xylylendiamin), 1,5-Pentandiisocyanat (aus 1,5-Pentandiamin), Hexamethylendiisocyanat (aus Hexamethylendiamin), Isophorondiisocyanat (aus Isophorondiamin) und Naphthyldiisocyanat (aus Naphthyldiamin), besonders bevorzugt von Methylen-Diphenylen-Diisocyanat, Gemischen aus Methylen-Diphenylen-Diisocyanat und Polymethylen-Polyphenylen-Polyisocyanat sowie Toluylendiisocyanat. Ganz besonders bevorzugt eignet sich das erfindungsgemäße Verfahren zur Herstellung von Methylen-Diphenylen-Diisocyanat und Gemischen aus Methylen-Diphenylen-Diisocyanat und Polymethylen-Polyphenylen-Polyisocyanat. Methylen-Diphenylen-Diisocyanat wird auch als Diisocyanat der Diphenylmethanreihe bezeichnet. Polymethylen-Polyphenylen-Polyisocyanat wird auch als Polisocyanat der Diphenylmethanreihe bezeichnet.

Verfahren zur Herstellung der genannten Amine sind dem Fachmann bekannt und bedürfen daher keine weitere Erläuterung an dieser Stelle.

In Schritt I) wird das zu phosgenierende Amin in einem Lösungsmittel gelöst. Dies kann mittels dem Fachmann bekannter Mischeinrichtungen wie insbesondere Mischrohren mit statischen Mischern als Einbauten (häufig auch kurz als *Statikmischer* bezeichnet) geschehen. Geeignete erfindungsgemäß einsetzbare *Lösungsmittel* sind dabei unter den Reaktionsbedingungen inerte Lösungsmittel wie z. B. Monochlorbenzol, Dichlorbenzol (insbesondere das *ortho*-Isomer), Dioxan, Toluol, Xylol, Methylenchlorid, Perchlorethylen, Trichlorfluormethan oder Butylacetat. Das Lösungsmittel ist bevorzugt im Wesentlichen frei von Isocyanat (Massenanteil < 100 ppm angestrebt) und im Wesentlichen frei von Phosgen (Massenanteil < 100 ppm angestrebt), worauf bei Einsatz von Recycleströmen zu achten ist. Bevorzugt wird daher nach einem Verfahren, wie in EP 1 854 783 A2 beschrieben, gearbeitet. Die Lösungsmittel können einzeln oder als beliebige Gemische der beispielhaft genannten Lösungsmittel eingesetzt werden. Vorzugsweise werden Monochlorbenzol (MCB) oder *ortho*-Dichlorbenzol (ODB) eingesetzt, ganz besonders bevorzugt Monochlorbenzol (MCB).

Vorgesehen ist eine Temperatur der resultierenden Amin-Lösung im Bereich von 30 °C bis 130 °C, insbesondere im Bereich von 60 °C bis 100 °C. Dies könnte prinzipiell erreicht werden durch entsprechende Temperierung der Ausgangsstoffe Amin und Lösungsmittel unter Berücksichtigung der Lösungsenthalpie. Es ist erfindungsgemäß jedoch vorgesehen, insbesondere zusätzlich zur genannten Temperierung der Ausgangsstoffe, einen der Vermischung von Amin und Lösungsmittel nachgeschalteten Wärmeaustauscher bereitzustellen, der die gezielte Einstellung der Amin-Lösung auf die gewünschte Temperatur im Bereich von 30 °C bis 130 °C, insbesondere im Bereich von 60 °C bis 100 °C, ermöglicht, der also je nachdem welche Temperatur unmittelbar nach der Vermischung der Ausgangsstoffe vorliegt, heizen oder kühlen kann. Hierzu eignen sich dem Fachmann bekannte Wärmeaustauscher wie insbesondere Rohrbündelwärmeaustauscher und Plattenwärmeaustauscher, die die Lösung durch indirekte Wärmeübertragung entsprechend temperieren. Die Wärmeaustauscher werden zu diesem Zweck mit geeigneten Wärmeträgermedien betrieben, insbesondere Öl, Salzschmelzen, organische Lösungsmittel (bevorzugt Monochlorbenzol, Dichlorbenzol oder Toluol, besonders bevorzugt Monochlorbenzol) oder - weniger bevorzugt, aber grundsätzlich auch möglich - (Heiz- bzw. Kühl-)Wasser. Die eingesetzten Wärmeträgermedien werden auf eine für die jeweilige Anforderung (Heizen oder Kühlen) geeignete Temperatur eingestellt. Hierzu geeignete Temperier-Vorrichtungen wie insbesondere Rohrbündelwärmeaustauscher, Plattenwärmeaustauscher und Spiralwärmeaustauscher sind dem Fachmann bekannt. Die Regelung der Betriebsweise eines solchen Wärmeaustauschers durch die Ist-Temperatur des Reaktionsgemisches aus Schritt (III) ist ein großer Vorteil der Erfindung.

Im Hinblick auf die Aminkonzentration in der in Schritt I) bereitgestellten Lösung ist es bevorzugt, den auf die Gesamtmasse dieser Lösung bezogenen Massenanteil an primärem Amin auf einen Wert im Bereich von 25 % bis 50 %, insbesondere im Bereich von 30 % bis 45 %, einzustellen. **Schritt II)** der vorliegenden Erfindung, die Bereitstellung der für die Phosgenierung erforderlichen Phosgenlösung, kann ebenfalls nach allen aus dem Stand der Technik bekannten Verfahren erfolgen. Es sind die gleichen Mischeinrichtungen und Lösungsmittel geeignet wie zuvor für das primäre Amin beschrieben. Insbesondere ist es bevorzugt, das primäre Amin in Schritt I) und Phosgen in Schritt II) jeweils im gleichen Lösungsmittel, ganz besonders bevorzugt also in MCB, zu lösen. Verfahren zur Herstellung von Phosgen sind dem Fachmann bekannt und bedürfen daher keine weitere Erläuterung an dieser Stelle.

Vorgesehen ist eine Temperatur der resultierenden Phosgen-Lösung im Bereich von -20 °C bis 120 °C, insbesondere im Bereich von -10 °C bis 30 °C. Dies könnte prinzipiell erreicht werden durch entsprechende Temperierung der Ausgangsstoffe Phosgen und Lösungsmittel unter Berücksichtigung der Lösungsenthalpie. Es ist erfindungsgemäß jedoch vorgesehen, insbesondere zusätzlich zur genannten Temperierung der Ausgangsstoffe, einen der Vermischung von Phosgen und Lösungsmittel nachgeschalteten Wärmeaustauscher bereitzustellen, der die gezielte Einstellung der Phosgen-Lösung auf die gewünschte Temperatur im Bereich von -20 °C bis 120 °C, insbesondere im Bereich von -10 °C bis 30 °C, ermöglicht, der also je nachdem welche Temperatur unmittelbar nach der Vermischung der Ausgangsstoffe vorliegt, heizen oder kühlen kann. Hierzu sind die gleichen Wärmeaustauscher geeignet wie zuvor für das primäre Amin beschrieben; die dort gemachten Ausführungen gelten an dieser Stelle daher ebenfalls. Bevorzugt wird in Schritt I) und in Schritt II) der gleiche Typ von Wärmeaustauscher unter Einsatz des gleichen Typs von Wärmeträgermedium eingesetzt. Die Regelung der Betriebsweise eines solchen Wärmeaustauschers durch die Ist-Temperatur des Reaktionsgemisches aus Schritt (III) ist ein großer Vorteil der Erfindung.

Im Hinblick auf die Phosgenkonzentration in der in Schritt II) bereitgestellten Lösung ist es bevorzugt, den auf die Gesamtmasse dieser Lösung bezogenen Massenanteil an Phosgen auf einen Wert im Bereich von 45 % bis 90 %, insbesondere im Bereich von 55 % bis 80 %, einzustellen.

In **Schritt III)** des erfindungsgemäßen Verfahrens erfolgt das Vermischen der in Schritt I) bereitgestellten Lösung des primären Amins und der in Schritt II) bereitgestellten Lösung. Hierzu eignen sich dem Fachmann bekannte Mischeinrichtungen wie statische oder dynamische Mischer. Statische Mischer sind durch die Abwesenheit beweglicher Teile gekennzeichnet, insbesondere seien hier Mischrohre mit statischen Mischern als Einbauten (häufig auch kurz als *Statikmischer* bezeichnet) oder Düsen genannt. Dynamische Mischer enthalten dagegen bewegliche Teile wie beispielsweise Rührorgane. Insbesondere sind hier auch die aus EP 0 830 894 A1 und EP 2 077 150 A1 bekannten Rotor-Stator-Systeme zu nennen. Dynamische Mischer, insbesondere solche vom Rotor-Stator-Typ, sind für den Einsatz in der vorliegenden Erfindung bevorzugt.

Bevorzugt wird die Mischeinrichtung aus Schritt III) nicht beheizt und nicht gekühlt, d. h. die Temperatur des erhaltenen Reaktionsgemisches wird allein durch die Mischungsenthalpie und die Enthalpie der bereits in der Mischeinrichtung einsetzenden Reaktionen bestimmt. Eine Transportleitung für das Reaktionsgemisch zwischen dem Austritt der Mischeinrichtung aus Schritt (III) und dem Eintritt in die Reaktionszone aus Schritt (IV) wird vorzugsweise ebenfalls weder beheizt noch gekühlt, ist jedoch vorzugsweise wärmeisoliert.

Erfindungsgemäß wird bei der Vermischung in Schritt III) ein auf die Aminogruppen des primären Amins bezogener stöchiometrischer Überschuss an Phosgen im Bereich von 40 % bis 200 % der Theorie, bevorzugt im Bereich von 40 % bis 120 % der Theorie, besonders bevorzugt im Bereich von 50 % bis 100 % der Theorie, ganz besonders bevorzugt im Bereich von 50 % bis 75 % der Theorie eingehalten.

In **Schritt IV)** des erfindungsgemäßen Verfahrens findet der erste Hauptteil der Reaktion zum Isocyanat statt, und zwar unter adiabatischen Bedingungen. Damit ist gemeint, dass die Schritt IV) durchlaufende Reaktionsmischung während der Umsetzung weder geheizt noch gekühlt wird. Die verwendeten Apparaturen sind gegen Wärmeverluste isoliert, sodass die Temperaturentwicklung von der Reaktionsenthalpie der ablaufenden Umsetzungen bestimmt wird.

Ohne auf eine Theorie festgelegt sein zu wollen ist davon auszugehen, dass in Schritt IV) mehrere Umsetzungen parallel ablaufen. Das primäre Amin reagiert mit Phosgen zur bekannten Zwischenstufe Carbaminsäurechlorid (exotherme Reaktion). Der dabei freigesetzte Chlorwasserstoff reagiert mit noch nicht umgesetzten Amin zu Aminhydrochlorid (exotherme Reaktion), das sich im eingesetzten Lösungsmittel löst (endotherme Reaktion). Partiell findet auch in Schritt IV) bereits die Spaltung des Carbaminsäurechlorids zum gewünschten Isocyanat und Chlorwasserstoff statt (endotherme Reaktion). Die Temperaturänderung hängt vom Zusammenspiel aller dieser Reaktionen statt. In der Regel wird in Schritt IV) nur eine geringe Temperaturänderung beobachtet, was dafür spricht, dass sich exo- und endotherme Reaktionen "die Waage halten". In jedem Fall bildet sich bei den Umsetzungen in Schritt IV) eine Gasphase aus, die in der Trennzone von der verbleibenden Flüssigphase getrennt wird. Reaktionszone und Trennzone sind bevorzugt in einem gemeinsamen Reaktor angeordnet. Hierzu eignen sich übliche, dem Fachmann bekannte Phosgenierungsreaktoren, wie insbesondere aufrecht angeordnete röhrenförmige Reaktoren (Rohrreaktoren; ist das Verhältnis von Höhe zu Durchmesser relativ klein, spricht man auch von Turmreaktoren oder Reaktortürmen), die bevorzugt mit dem in Schritt III) erhaltenen Reaktionsgemisch von unten nach oben durchströmt werden. Zur Einengung der Verweilzeitverteilung können die Reaktoren in der Reaktionszone durch dem Fachmann bekannte Einbauten segmentiert sein. Im oberen Teil des Reaktors werden die gebildete Gasphase und die verbleibende Flüssigphase getrennt entnommen. Die Phasentrennung findet spontan statt.

Erfindungsgemäß wird für die Temperatur des Reaktionsgemisches aus Schritt III) ein Soll-Wert im Bereich von 110 °C bis 145 °C festgelegt. Ein (im Sinne minimierter Nebenproduktbildung und minimierten Lösungsmitteleinsatzes) geeigneter Soll-Wert ist abhängig vom herzustellenden Isocyanat und Anlagenparametern und kann vom Fachmann für eine gegebenen Produktionsanlage leicht durch Vorversuche ermittelt werden. Die tatsächlich vorliegende Temperatur (Ist-Temperatur) kann dabei mit dem Fachmann bekannten Temperaturmessfühlern, die an geeigneter Stelle (insbesondere am Austritt der Mischeinrichtung aus Schritt III) angebracht sind, gemessen werden.

Die erfindungsgemäße Einstellung der Temperatur in der Reaktionszone und Trennzone erfolgt nun, indem man die Temperatur des die Mischeinrichtung aus Schritt III) verlassenden Reaktionsgemisches misst und mit einem zuvor festgelegten Soll-Wert innerhalb des Bereichs von 110 °C bis 145 °C vergleicht und festgestellte Abweichungen durch Erhöhen oder Erniedrigen der Temperatur der in Schritt I) bereitgestellten Lösung des primären Amins und/oder der Temperatur der in Schritt II) bereitgestellten Lösung von Phosgen ausgleicht.

Hierzu werden die weiter oben erwähnten der Vermischung von Amin und Lösungsmittel und der Vermischung von Phosgen und Lösungsmittel jeweils nachgeschalteten Wärmeaustauscher eingesetzt. Erfindungsgemäß werden also festgestellte Abweichungen vom Soll-Wert der Temperatur des die Mischeinrichtung aus Schritt III) verlassenden Reaktionsgemisches durch Erhöhen oder Erniedrigen der Temperatur der in Schritt I) bereitgestellten Lösung des primären Amins und/oder der in Schritt II) bereitgestellten Lösung von Phosgen ausgeglichen, wobei die in Schritt I) bereitgestellte Lösung des primären Amins und die in Schritt II) bereitgestellte Lösung von Phosgen der Mischeinrichtung aus Schritt III) über jeweils einen Wärmeaustauscher zugeführt werden, und wobei das Erhöhen oder Erniedrigen der Temperatur der in Schritt I) bereitgestellten Lösung des primären Amins und/oder der in Schritt II) bereitgestellten Lösung von Phosgen durch entsprechende Änderung der Betriebsweise des jeweiligen Wärmeaustauschers (verstärktes Heizen, verstärktes Kühlen oder sogar Umkehrung des Heiz- in den Kühlmodus oder des Kühl- in den Heizmodus) bewirkt wird.

Hierbei ist folgende automatisierte Vorgehensweise für Schritt IV) besonders bevorzugt:

- Eintragen des Soll-Werts für die Temperatur des Reaktionsgemisches aus Schritt III) in eine elektronische Datenverarbeitungsanlage;
- Bereitstellen der Messwerte der Ist-Temperatur des Reaktionsgemisches aus Schritt III) in elektronischer Form;
- Übermitteln der in elektronischer Form bereitgestellten Messwerte an die elektronische Datenverarbeitungsanlage;
- Abgleichen des Soll-Werts mit der Ist-Temperatur in der elektronischen Datenverarbeitungsanlage;
- bei festgestellter Abweichung der Ist-Temperatur vom Soll-Wert um mehr als 1,0 °C (bevorzugt um mehr als 0,5 °C):

∘ bei Abweichung nach oben, Reduzieren
∘ bei Abweichung nach unten, Erhöhen

der Temperatur des in den Wärmeaustauschern verwendeten Wärmeträgermediums durch Übermitteln eines entsprechenden Befehls aus der elektronischen Datenverarbeitungsanlage an die zur Temperierung des Wärmeträgermediums eingesetzte Temperier-Vorrichtung.

Geeignete Hard- und Software für die Umsetzung dieser Ausführungsform ist dem Fachmann bekannt.

In **Schritt V)** des erfindungsgemäßen Verfahrens wird das in Schritt IV) erhaltene flüssige Verfahrensprodukt auf einen geringeren Druck entspannt, und zwar bevorzugt auf einen Druck im Bereich von 1,0 bar$_{(abs.)}$ bis 20 bar$_{(abs.)}$, bevorzugt 1,0 bar$_{(abs.)}$ bis 5,0 bar$_{(abs.)}$, gemessen in der Gasphase. Hierzu eignen sich dem Fachmann bekannte Apparaturen wie insbesondere Gas-Flüssigkeits-Trennbehälter (auch als *Gasabscheider* bezeichnet). Dabei bildet sich eine Chlorwasserstoff und nicht umgesetztes Phosgen enthaltende Gasphase aus. Die eingesetzte Apparatur kann auch im gleichen Apparat wie die indirekt beheizte Reaktionszone aus Schritt VI) angeordnet werden; siehe hierzu auch die folgenden Erläuterungen zu Schritt VI).

In **Schritt VI)** wird die in Schritt IV) nach der Entspannung verbleibende Flüssigphase in einer indirekt beheizten Reaktionszone unter Ausbildung einer Chlorwasserstoff- und Phosgen-haltigen Gasphase weiter umgesetzt ("isotherme Verfahrensführung"). Dies kann in dem Fachmann bekannten heizbaren Reaktoren stattfinden. Insbesondere geeignet sind zu diesem Zweck (vertikal angeordnete) Rohrbündelreaktoren. Dabei kann die Flüssigphase aus Schritt IV) durch das Innere der Rohre des Rohrbündelreaktors (Rohrinnenraum) oder durch den Raum zwischen den Rohren des Rohrbündelreaktors, der nach außen durch die das Rohrbündel umhüllende Reaktorwand begrenzt wird (Rohraußenraum), geführt werden. Das Heizmedium - ein Wärmeträgeröl, eine Salzschmelze, Dampf oder dergleichen - wird dann durch den jeweils anderen Raum geführt, sodass es nicht in stofflichen Kontakt mit dem umzusetzenden flüssigen Verfahrensprodukt tritt (indirekte Beheizung). Die Flüssigphase aus der Entspannung von Schritt IV) durchläuft dabei den vertikal angeordneten Rohrbündelreaktor vorzugsweise von oben nach unten. In der bevorzugten Ausgestaltung der Erfindung unter Integration der für die Entspannungsstufe von Schritt IV) vorgesehenen Apparatur in den die indirekt beheizte Reaktionszone enthaltenden Apparat aus Schritt V) findet dann die Entspannung unter Gas-Flüssig-Phasentrennung in einer Haube am Kopf des Rohrbündelreaktors statt.

In den Schritten IV), V) und VI) fallen Gasphasen enthaltend Chlorwasserstoff und Phosgen sowie gegebenenfalls Lösungsmittel an. Bevorzugt werden diese Gasphasen zur Rückgewinnung von Wertprodukten aufgearbeitet. Hierzu ist es zweckmäßig, die anfallenden Gasphasen vor der Aufarbeitung auf einen gemeinsamen Druck einzustellen und zu vereinigen. Diese Aufarbeitung kann geschehen wie im Stand der Technik bekannt.

Durch das erfindungsgemäße Verfahren ergeben sich mindestens die folgenden Vorteile ohne die Produktqualität hinsichtlich Nebenkomponentenbildung, Farbe, Acidität, NCO-Gehalt und Eisengehalt zu beeinträchtigen:

i) Verbesserung der Energieeffizienz des Verfahrens durch Optimierung der Rezeptur hinsichtlich des Verhältnisses von Lösungsmittel zu Amin (Verringerung der Menge zu rezyklierenden Lösungsmittels);
ii) Die Regelung über die Temperatur der Reaktionsmischung aus Schritt III) ist einfach durchzuführen und ermöglicht eine bessere Qualitätskontrolle (die Farbe des Produkts und der Nebenkomponenten-Gehalt lassen sich einfach auf akzeptablen Werten halten; dadurch besteht auch die Möglichkeit, in der sog. Polymerabtrennung - der destillativen Abtrennung einer Fraktion enthaltend Methylen-Diphenylen-Diisocyanat aus einem Gemisch enthaltend Polymethylen-Polyphenylen-Polyisocyanat und Methylen-Diphenylen-Diisocyanat - mehr Methylen-Diphenylen-Diisocyanat abzudestillieren, ohne die Zusammensetzung des angestrebten Polymer-Typs zu verändern [da die Ausbeute an Monomer-MDI infolge verringerter Nebenproduktbildung erhöht ist]).

Der Erfolg der erfindungsgemäßen Vorgehensweise zur Steuerung der Phosgenierungs-Reaktion bei "adiabatisch-isothermer Betriebsweise" mittels Temperaturregelung der Einsatzströme von Amin- und/oder Phosgen-Lösung war für den Fachmann überraschend, da über Komplikationen mit der Temperaturerhöhung bei der Phosgenierung infolge exothermer Reaktionen (*die ja bereits in der Mischeinrichtung aus Schritt* III) *auftreten*) bisher nicht berichtet wurde. Mit der beschriebenen Temperaturregelung sind besonders lösungsmittelarme Phosgenierungsreaktionen möglich. Es wird dann weniger Energie zur Rezyklierung von überschüssigem Lösungsmittel verbraucht und die Produktqualität gesteigert.

Nachfolgend wird die Erfindung anhand von Beispielen näher erläutert.

## Beispiele:

### Allgemeine Arbeitsvorschrift für die Durchführung der Bespiele 1 bis 4

MDA und MCB werden kontinuierlich zu einer 30%igen MDA-Lösung vermischt. Ebenso werden Phosgen und MCB kontinuierlich zu einer 60%igen Phosgenlösung vermischt. Die Temperaturen der erhaltenen MDA- und Phosgen-Lösungen werden jeweils durch einen Wärmeaustauscher eingestellt. Die MDA-Lösung (64 kg/h) und die Phosgenlösung (51 kg/h) werden dynamisch (Stachelmischer) kontinuierlich vermischt, wobei der Phosgenüberschuss jeweils 60 % der Theorie beträgt. Der Druck im Stachelmischer beträgt 22,0 bar$_{(abs.)}$. Die Austrittstemperatur des Reaktionsgemisches hinter dem Stachelmischer stellt sich infolge des Wechselspiels von exo- und endothermen Reaktionen ein, hängt also vom unter den jeweiligen Bedingungen vorliegenden Reaktionsfortschritt ab. Das den Stachelmischer verlassende Reaktionsgemisch wird unter dem im Wesentlichen gleichen Druck (also 22,0 bar$_{(abs.)}$ zzgl. einem vergleichsweise kleinen Anteil des hydrostatischen Drucks) durch einen ersten, weder geheizten noch gekühlten, aber gegen Wärmeverluste isolierten, aufrecht angeordneten Rohrreaktor R1 geführt. Nach einer Verweilzeit von 2,0 min werden dem Reaktor R1 im oberen Bereich (Trennzone) eine Gasphase und eine Flüssigphase an unterschiedlichen Stellen entnommen. Die Temperatur der entnommenen Flüssigphase am Austritt aus R1 ist bei den gewählten Reaktionsbedingungen nur wenig von der Eintrittstemperatur verschieden (Abweichung $\pm$ 5,0 °C), was darauf hindeutet, dass sich exo- und endotherme Reaktionen im Reaktor R1 in etwa die Waage halten. Die Flüssigphase wird in einem Gasabscheider auf einen Druck von 2,0 bar$_{(abs.)}$ entspannt, und die den Gasabscheider verlassende Flüssigphase wird durch einen zweiten, indirekt beheizten Rohrreaktor R2 geführt. Die Verweilzeit im zweiten Reaktor R2 wird so gewählt, dass die Spaltung von Carbaminsäurechlorid nahezu vollständig ist. Die den zweiten Reaktor verlassende Flüssigphase wird entsprechend dem Stand der Technik von Phosgen, Chlorwasserstoff und MCB befreit.

### Beispiele 1 bis 4

Die Temperaturen der Amin- und Phosgenlösung wurden variiert, um den Einfluss auf die Qualität des erhaltenen Produkts (Farbwerte, NCO-Index und Acidität) zu untersuchen. Die Betriebsbedingungen können Tabelle 1 entnommen werden.

Wie man sieht, sind die Farbwerte, der NCO-Index und der Aciditätswert insgesamt dann am besten, wenn die Ausgangstemperaturen von Amin- **und** Phosgenlösung (welche bei gegebenen Randbedingungen die Mischeraustrittstemperatur bestimmen) niedrig sind (Beispiel 4). Dies zeigt die große Bedeutung des erfindungsgemäßen Verfahrens, welches es ermöglicht, im Falle von unerwünschten Abweichungen der Mischeraustrittstemperatur auf einfache Weise schnell gegenzusteuern und auf diese Weise die Temperatur im Reaktor von Schritt IV) einzustellen.

**Tabelle 1: Betriebsbedingungen und Ergebnisse in den Beispielen 1 bis 4**

| Beispiel | T(MDA-Lsg.) / °C | T(COCl$_2$)-Lsg. / °C | T(Austritt Mischer) / °C | Gelbwert E430 | Grauwert E520 | NCO-Wert / % | Acidität / ppm |
|---|---|---|---|---|---|---|---|
| 1 | 120 | -2,0 | 141,0 | 0,131 | 0,032 | 30,6 | 403 |
| 2 | 100 | 63 | 139,0 | 0,306 | 0,115 | 30,6 | 200 |
| 3 | 60 | 80 | 139,0 | 0,281 | 0,102 | 30,9 | 120 |
| 4 | 60 | -2,0 | 129,0 | 0,108 | 0,036 | 31,2 | 39 |

Analysenmethoden:
Farbe (Gelbwert, Grauwert): Herstellung einer ca. 2%igen Lösung (massebezogen) der Probe in Monochlorbenzol, Bestimmung der Extinktion bei 430 nm (E430,G$^{elbwert}$) und bei 520 nm (E520, Grauwert) mit Hilfe eines UV/VIS Spektralphotometers unter Verwendung einer Küvette mit einer Schichtdicke von 1 cm.
NCO-Wert: Umsetzung mit Dibutylamin und Rücktitration des nicht umgesetzten Dibutylamins mit einer HCl-Maßlösung.
Acidität: Lösen von ca. 6 g der Probe in 40 ml Monochlorbenzol, Zugabe von 100 ml Methanol und Rühren bei Raumtemperatur für 30 min. Titration mit.$^{methanolischer}$ KOH-Maßlösung.

**Beispiel 5 (erfindungsgemäß): Computer-Simulation des erfindungsgemäßen Verfahrens für Produktion im industriellen Maßstab.**

In einer MDI-Anlage werden 40,0 t/h MDA mit einer Temperatur von 130 °C mit 94,5 t/h MCB einer Temperatur von 52 °C als Lösungsmittel über einen statischen Mischer zu einer 30,0%igen MDA-Lösung vermischt (**Schritt I)**). Phosgen wird in einem Phosgenlösungstank mit MCB unter Erhalt einer 60,0%igen Phosgenlösung vermischt (**Schritt II)**). Stündlich werden 106,7 Tonnen dieser Phosgenlösung einer Temperatur von 3,0 °C durch einen Wärmeaustauscher geführt und so auf eine Temperatur von -1 °C abgekühlt. Analog werden stündlich 134,5 Tonnen der 30,0%igen MDA-Lösung einer Temperatur von 80,0 °C durch einen Wärmeaustauscher geführt und so auf eine Temperatur von 60 °C abgekühlt. Die so temperierten MDA- und Phosgenlösungen werden in einen dynamischen Mischer geführt (**Schritt III)**). Die Temperatur am Austritt des dynamischen Mischers wird auf 130 °C eingestellt.

Die den Mischer verlassende flüssige Reaktionsmischung wird unter adiabatischen Bedingungen durch einen gegen Wärmeverluste isolierten Phosgenierreaktor (Turmreaktor) geführt (**Schritt IV)**). Der Druck am Austritt des Phosgenierreaktors wird mittels eines Druckhalteventils auf 22 bar$_{(abs.)}$ eingestellt; die Austrittstemperatur beträgt 120 °C. Die Verweilzeit der Phosgenierungsreaktion vom Mischer bis zum Austritt aus dem Phosgenierreaktors beträgt 5 min. Im Kopf des Turmreaktors scheidet sich eine HCl- und Phosgen-haltige Gasphase (die auch noch Anteile an verdampftem MCB enthält) ab. Die dem Reaktor entnommene Reaktionslösung wird in einem Gasabscheider auf 3 bar$_{(abs.)}$ entspannt (**Schritt V)**) und anschließend in einem beheizten Reaktor bei 130 °C und 3 bar$_{(abs.)}$ weiter umgesetzt (**Schritt VI)**).

Anschließend wird die den beheizten Reaktor verlassende Reaktionslösung unter Rückgewinnung des Lösungsmittels und Erhalt des Isocyanats aufgearbeitet (**Schritt VII)**). Die Aufarbeitung umfasst eine Entphosgenierung und Lösungsmittelabtrennung. In der Destillation zur Lösungsmittelabtrennung fallen als Sumpfprodukt 50,0 t/h MDI an, das mittels weiterer Destillationsschritte in Methylen-Diphenylen-Diisoyanat und ein Gemisch aus Methylen-Diphenylen-Diisoyanat und Polymethylen-Polyphenylen-Polyisocyanat getrennt wird.

Die Einstellung der Temperatur im Turmreaktor erfolgt durch Messung der Mischeraustrittstemperatur und Regelung der Temperatur der MDA- bzw. Phosgenlösung derart, dass bei signifikanten Abweichung von der Soll-Temperatur 130 °C die Betriebsweise der entsprechenden Wärmeaustauscher so geändert wird, dass der Zielwert von 130 °C möglichst rasch wieder erreicht wird. Die beiden Wärmeaustauscher werden also durch die Temperatur am Austritt des Mischers geregelt, was bedeutet, dass sie nach Bedarf beheizt oder gekühlt werden, wodurch sich die Temperaturen der Phosgen- und MDA-Lösung, die zur Erreichung der angestrebten Mischeraustrittstemperatur von 130 °C erforderlich sind, einstellen. Durch diese Vorgehensweise wird die Temperatur am Austritt des Phosgenierreaktors konstant auf 120 °C gehalten.

In einer realen Produktionsanlage können die hier genannten Mengenströme vorteilhafterweise in mehreren parallel betriebenen Reaktionsstraßen umgesetzt werden.

**Patentansprüche**

1. Verfahren zur Herstellung eines Isocyanats durch Umsetzung eines primären Amins mit Phosgen, umfassend die Schritte:

   I) Bereitstellen einer Lösung des primären Amins in einem Lösungsmittel und Temperieren der Lösung des primären Amins durch indirekte Wärmeübertragung in einem mit einem Wärmeträgermedium betriebenen Wärmeaustauscher;
   II) Bereitstellen einer Lösung von Phosgen in einem Lösungsmittel und Temperieren der Lösung von Phosgen durch indirekte Wärmeübertragung in einem mit einem Wärmeträgermedium betriebenen Wärmeaustauscher;
   III) Vermischen der in Schritt I) bereitgestellten Lösung des primären Amins und der in Schritt II) bereitgestellten Lösung von Phosgen in einer Mischeinrichtung zu einem Reaktionsgemisch unter Einhaltung eines auf die Aminogruppen des primären Amins bezogenen stöchiometrischen Überschusses an Phosgen im Bereich von 40 % bis 200 % der Theorie;
   IV) Führen des in Schritt III) erhaltenen Reaktionsgemisches durch eine Reaktionszone und durch eine dieser Reaktionszone strömungstechnisch nachgeschaltete Trennzone unter Ausbildung einer unter einem Druck im Bereich von 8,0 bar$_{(abs.)}$ bis 50,0 bar$_{(abs.)}$ stehenden Gasphase aus dem flüssigen Reaktionsgemisch in der Trennzone, wobei die Reaktionszone und die Trennzone nicht beheizt und nicht gekühlt werden, wobei die in der Trennzone gebildete Gasphase und die verbleibende Flüssigphase der Trennzone getrennt voneinander entnommen werden;
   wobei man die Temperatur in der Reaktionszone und Trennzone einstellt, indem man für die Temperatur des Reaktionsgemisches aus Schritt III) einen Soll-Wert innerhalb eines Bereiches von 110 °C bis 145 °C festlegt und die kontinuierlich oder in Intervallen gemessene Ist-Temperatur des Reaktionsgemisches aus Schritt III)

zur Regelung

der Temperatur der in Schritt I) bereitgestellten Lösung des primären Amins auf einen Wert im Bereich von 30 °C bis 130 °C mittels des in Schritt I) eingesetzten Wärmemaustauschers
und/oder
der Temperatur der in Schritt II) bereitgestellten Lösung von Phosgen auf einen Wert im Bereich von -20 °C bis 120 °C mittels des in Schritt II) eingesetzten Wärmeaustauschers
verwendet;

V) Entspannen der der Trennzone aus Schritt IV) entnommenen Flüssigphase unter partieller Überführung dieser Flüssigphase in die Gasphase;
VI) Führen der in Schritt V) nach dem Entspannen verbleibenden Flüssigphase durch eine indirekt beheizte Reaktionszone, wobei sich eine Chlorwasserstoff- und Phosgen-haltige Gasphase ausbildet, die abgetrennt wird, und eine Isocyanat- und Lösungsmittel enthaltende Flüssigphase verbleibt, die der indirekt beheizten Reaktionszone entnommen wird;
VII) Aufarbeiten der in Schritt VI) gewonnenen Isocyanat- und Lösungsmittel enthaltenden Flüssigphase unter Rückgewinnung des Lösungsmittels und Erhalt des Isocyanats.

2. Verfahren gemäß Anspruch 1, bei welchem die in Schritt I) bereitgestellte Lösung des primären Amins einen auf die Gesamtmasse dieser Lösung bezogenen Massenanteil an primärem Amin im Bereich von 25 % bis 50 % und die in Schritt II) bereitgestellte Lösung von Phosgen einen auf die Gesamtmasse dieser Lösung bezogenen Massenanteil an Phosgen im Bereich von 45 % bis 90 % aufweist.

3. Verfahren gemäß Anspruch 1 oder 2, bei welchem die in den Schritten IV), V) und VI) anfallenden Gasphasen zur Gewinnung von Chlorwasserstoff und Phosgen sowie gegebenenfalls Lösungsmittel aufgearbeitet werden.

4. Verfahren gemäß Anspruch 3, bei welchem die in den Schritten IV), V) und VI) anfallenden Gasphasen vor der Aufarbeitung auf einen gemeinsamen Druck eingestellt und vereinigt werden.

5. Verfahren gemäß einem der vorstehenden Ansprüche, bei welchem die Mischeinrichtung aus Schritt III) nicht beheizt und nicht gekühlt wird.

6. Verfahren gemäß einem der vorstehenden Ansprüche, bei welchem die in Schritt III) eingesetzte Mischeinrichtung einen oder mehrere dynamischen Mischer umfasst.

7. Verfahren gemäß einem der vorstehenden Ansprüche, bei welchem Reaktionszone und Trennzone aus Schritt IV) in einem gemeinsamen Reaktor angeordnet sind.

8. Verfahren gemäß Anspruch 7, bei welchem der Reaktor ein aufrecht angeordneter röhrenförmiger Reaktor ist.

9. Verfahren gemäß Anspruch 8, bei welchem der Reaktor mit dem in Schritt III) erhaltenen Reaktionsgemisch von unten nach oben durchströmt wird.

10. Verfahren gemäß einem der vorstehenden Ansprüche, bei welchem die indirekt beheizte Reaktionszone aus Schritt VI) Bestandteil eines Rohrbündelreaktors ist, durch dessen Rohrinnenraum die in Schritt V) nach dem Entspannen verbleibende Flüssigphase geführt wird und durch dessen Rohraußenraum ein Heizmedium geführt wird, oder durch dessen Rohraußenraum die in Schritt V) nach dem Entspannen verbleibende Flüssigphase geführt wird und durch dessen Rohrinnenraum ein Heizmedium geführt wird.

11. Verfahren gemäß einem der vorstehenden Ansprüche, bei welchem die in Schritt I) und in Schritt II) eingesetzten Wärmeaustauscher unabhängig voneinander ausgewählt sind aus der Gruppe bestehend aus Rohrbündelwärmeaustauschern und Plattenwärmeaustauschern.

12. Verfahren gemäß einem der vorstehenden Ansprüche, bei welchem die in Schritt I) und in Schritt II) eingesetzten Wärmeaustauscher unabhängig voneinander mit einem Wärmeträgermedium ausgewählt aus der Gruppe bestehend aus Öl, Salzschmelzen, organischen Lösungsmitteln und Wasser betrieben werden.

13. Verfahren gemäß einem der vorstehenden Ansprüche, bei welchem in der Trennzone aus Schritt IV) ein Druckhalteventil für die sich ausbildende Gasphase und eine Flüssigkeitsstandregelung für die Flüssigphase angeordnet sind, durch welche der Druck der Gasphase konstant gehalten wird.

**14.** Verfahren gemäß einem der vorstehenden Ansprüche, bei welchem Schritt IV) umfasst:

• Eintragen des Soll-Werts für die Temperatur des Reaktionsgemisches aus Schritt III) in eine elektronische Datenverarbeitungsanlage;
• Bereitstellen der Messwerte der Ist-Temperatur des Reaktionsgemisches aus Schritt III) in elektronischer Form;
• Übermitteln der in elektronischer Form bereitgestellten Messwerte an die elektronische Datenverarbeitungsanlage;
• Abgleichen des Soll-Werts mit der Ist-Temperatur in der elektronischen Datenverarbeitungsanlage;
• bei festgestellter Abweichung der Ist-Temperatur vom Soll-Wert um mehr als 1,0 °C:

∘ bei Abweichung nach oben, Reduzieren
∘ bei Abweichung nach unten, Erhöhen

der Temperatur des in den Wärmeaustauschern verwendeten Wärmeträgermediums durch Übermitteln eines entsprechenden Befehls aus der elektronischen Datenverarbeitungsanlage an die zur Temperierung des Wärmeträgermediums eingesetzte Temperier-Vorrichtung.

**15.** Verfahren gemäß einem der vorstehenden Ansprüche, bei welchem

(i) Methylen-Diphenylen-Diisocyanat und/oder Polymethylen-Polyphenylen-Polyisocyanat durch Umsetzung von Methylen-Diphenylen-Diamin und/oder Polymethylen-Polyphenylen-Polyamin mit Phosgen oder
(ii) Toluylendiisocyanat durch Umsetzung von Toluylendiamin mit Phosgen hergestellt wird.

**Claims**

**1.** Process for preparing an isocyanate by reacting a primary amine with phosgene, comprising the steps of:

I) providing a solution of the primary amine in a solvent and adjusting the temperature of the solution of the primary amine by indirect heat transfer in a heat exchanger operated with a heat carrier medium;
II) providing a solution of phosgene in a solvent and adjusting the temperature of the solution of phosgene by indirect heat transfer in a heat exchanger operated with a heat carrier medium;
III) mixing the solution of the primary amine provided in step I) and the solution of phosgene provided in step II) in a mixing unit to give a reaction mixture with observance of a stoichiometric excess of phosgene based on the amino groups of the primary amine in the range from 40% to 200% of theory;
IV) running the reaction mixture obtained in step III) through a reaction zone and through a separation zone downstream in flow direction of this reaction zone to form a gas phase under a pressure in the range from 8.0 $bar_{(abs.)}$ to 50.0 $bar_{(abs.)}$ from the liquid reaction mixture in the separation zone, where the reaction zone and the separation zone are not heated and not cooled, where the gas phase formed in the separation zone and the remaining liquid phase from the separation zone are removed separately from one another;

where the temperature in the reaction zone and separation zone is adjusted by fixing a target value within a range from 110°C to 145°C for the temperature of the reaction mixture from step III) and using the actual temperature of the reaction mixture from step III) measured continuously or at intervals for closed-loop control of the temperature of the solution of the primary amine provided in step I) to a value in the range from 30°C to 130°C by means of the heat exchanger used in step I)
and/or
of the temperature of the solution of phosgene provided in step II) to a value in the range from -20°C to 120°C by means of the heat exchanger used in step II);

V) expanding the liquid phase withdrawn from the separation zone from step IV) with partial conversion of this liquid phase to the gas phase;
VI) running the liquid phase that remains after the expansion in step V) through an indirectly heated reaction zone, forming a hydrogen chloride- and phosgene-containing gas phase which is removed, and an isocyanate- and solvent-containing liquid phase remaining which is withdrawn from the indirectly heated reaction zone;

VII) working up the isocyanate- and solvent-containing liquid phase obtained in step VI) to recover the solvent and obtain the isocyanate.

2. Process according to Claim 1, in which the solution of the primary amine provided in step I) has a proportion by mass of primary amine based on the total mass of this solution in the range from 25% to 50% and the solution of phosgene provided in step II) has a proportion by mass of phosgene based on the total mass of this solution in the range from 45% to 90%.

3. Process according to Claim 1 or 2, in which the gas phases obtained in steps IV), V) and VI) are worked up to obtain hydrogen chloride and phosgene and optionally solvent.

4. Process according to Claim 3, in which the gas phases obtained in steps IV), V) and VI) are prior to the workup adjusted to a common pressure and combined.

5. Process according to any of the preceding claims, in which the mixing unit from step III) is not heated and not cooled.

6. Process according to any of the preceding claims, in which the mixing unit used in step III) comprises one or more dynamic mixers.

7. Process according to any of the preceding claims, in which reaction zone and separation zone from step IV) are disposed in a common reactor.

8. Process according to Claim 7, in which the reactor is a tubular reactor in an upright arrangement.

9. Process according to Claim 8, in which the reaction mixture obtained in step III) flows through the reactor from the bottom upward.

10. Process according to any of the preceding claims, in which the indirectly heated reaction zone from step VI) is part of a shell and tube reactor, the liquid phase remaining after the expansion in step V) is run through the tube interior thereof and a heating medium is run through the tube exterior thereof, or the liquid phase remaining after the expansion in step V) is run through the tube exterior thereof and a heating medium is run through the tube interior thereof.

11. Process according to any of the preceding claims, in which the heat exchangers used in step I) and in step II) are independently selected from the group consisting of shell and tube heat exchangers and plate heat exchangers.

12. Process according to any of the preceding claims, in which the heat exchangers used in step I) and in step II) are independently operated with a heat carrier medium selected from the group consisting of oil, salt melts, organic solvents and water.

13. Process according to any of the preceding claims, in which a pressure-retaining valve for the gas phase that forms and a closed-loop liquid level controller for the liquid phase are disposed in the separation zone from step IV), by means of which the pressure of the gas phase is kept constant.

14. Process according to any of the preceding claims, in which step IV) comprises:

☐ entering the target value for the temperature of the reaction mixture from step III) into an electronic data processing system;
☐ providing the measured values of the actual temperature of the reaction mixture from step III) in electronic form;
☐ transmitting the measured values provided in electronic form to the electronic data processing system;
☐ comparing the target value with the actual temperature in the electronic data processing system;
☐ if a deviation in the actual temperature from the target value by more than 1.0°C is found:

   ∘ in the event of an upward deviation reducing
   ∘ in the event of a downward deviation increasing the temperature of the heat carrier medium used in the heat exchangers by transmitting a corresponding command from the electronic data processing system to the temperature control device used to adjust the temperature of the heat carrier medium.

**15.** Process according to any of the preceding claims, in which

(i) methylene diphenylene diisocyanate and/or polymethylene polyphenylene polyisocyanate is prepared by reacting methylene diphenylene diamine and/or polymethylene polyphenylene polyamine with phosgene or
(ii) tolylene diisocyanate is prepared by reacting tolylenediamine with phosgene.

**Revendications**

**1.** Procédé de fabrication d'un isocyanate par mise en réaction d'une amine primaire avec du phosgène, comprenant les étapes suivantes :

I) la préparation d'une solution de l'amine primaire dans un solvant et le conditionnement en température de la solution de l'amine primaire par transfert indirect de chaleur dans un échangeur de chaleur exploité avec un milieu caloporteur ;
II) la préparation d'une solution de phosgène dans un solvant et le conditionnement en température de la solution de phosgène par transfert indirect de chaleur dans un échangeur de chaleur exploité avec un milieu caloporteur ;
III) le mélange de la solution de l'amine primaire préparée dans l'étape I) et de la solution de phosgène préparée dans l'étape II) dans un appareil de mélange en un mélange réactionnel avec conservation d'un excès stœchiométrique de phosgène rapporté aux groupes amino de l'amine primaire dans la plage allant de 40 % à 200 % de la théorie ;
IV) l'acheminement du mélange réactionnel obtenu dans l'étape III) à travers une zone de réaction et à travers une zone de séparation raccordée fluidiquement en aval de cette zone de réaction avec formation d'une phase gazeuse se trouvant sous une pression dans la plage allant de 8,0 bar$_{(abs.)}$ à 50,0 bar$_{(abs.)}$ à partir du mélange réactionnel liquide dans la zone de séparation, la zone de réaction et la zone de séparation n'étant pas chauffées et n'étant pas refroidies, la phase gazeuse formée dans la zone de séparation et la phase liquide restante étant soutirées séparément l'une de l'autre de la zone de séparation ;
la température dans la zone de réaction et la zone de séparation étant ajustée en établissant une valeur de consigne dans une plage allant de 110 °C à 145 °C pour la température du mélange réactionnel de l'étape III) et en utilisant la température réelle du mélange réactionnel de l'étape III) mesurée continuellement ou par intervalles pour la régulation

de la température de la solution de l'amine primaire préparée dans l'étape I) à une valeur dans la plage allant de 30 °C à 130 °C au moyen de l'échangeur de chaleur utilisé dans l'étape I),
et/ou
de la température de la solution de phosgène préparée dans l'étape II) à une valeur dans la plage allant de -20 °C à 120 °C au moyen de l'échangeur de chaleur utilisé dans l'étape II) ;

V) la détente de la phase liquide soutirée de la zone de séparation de l'étape IV) avec passage partiel de cette phase liquide dans la phase gazeuse ;
VI) l'acheminement de la phase liquide restante après la détente dans l'étape V) à travers une zone de réaction chauffée indirectement, une phase gazeuse contenant du chlorure d'hydrogène et du phosgène se formant, qui est séparée, et une phase liquide contenant de l'isocyanate et du solvant restant, qui est soutirée de la zone de réaction chauffée indirectement ;
VII) le traitement de la phase liquide contenant de l'isocyanate et du solvant obtenue dans l'étape VI) avec récupération du solvant et obtention de l'isocyanate.

**2.** Procédé selon la revendication 1, selon lequel la solution de l'amine primaire préparée dans l'étape I) présente une proportion massique d'amine primaire rapportée à la masse totale de cette solution dans la plage allant de 25 % à 50 % et la solution de phosgène préparée dans l'étape II) présente une proportion massique de phosgène rapportée à la masse totale de cette solution dans la plage allant de 45 % à 90 %.

**3.** Procédé selon la revendication 1 ou 2, selon lequel les phases gazeuses formées dans les étapes IV), V) et VI) sont traitées pour l'obtention de chlorure d'hydrogène et de phosgène, ainsi qu'éventuellement de solvant.

**4.** Procédé selon la revendication 3, selon lequel les phase gazeuses formées dans les étapes IV), V) et VI) sont ajustées à une pression commune et réunies avant le traitement.

**5.** Procédé selon l'une quelconque des revendications précédentes, selon lequel l'appareil de mélange de l'étape III) n'est pas chauffé et n'est pas refroidi.

**6.** Procédé selon l'une quelconque des revendications précédentes, selon lequel l'appareil de mélange utilisé dans l'étape III) comporte un ou plusieurs mélangeurs dynamiques.

**7.** Procédé selon l'une quelconque des revendications précédentes, selon lequel la zone de réaction et la zone de séparation de l'étape IV) sont agencées dans un réacteur commun.

**8.** Procédé selon la revendication 7, selon lequel le réacteur est un réacteur de forme tubulaire agencé verticalement.

**9.** Procédé selon la revendication 8, selon lequel le réacteur est traversé du bas vers le haut par le mélange réactionnel obtenu dans l'étape III).

**10.** Procédé selon l'une quelconque des revendications précédentes, selon lequel la zone de réaction chauffée indirectement de l'étape VI) fait partie d'un réacteur à faisceau de tubes, à travers l'espace intérieur de tubes duquel la phase liquide restante après la détente dans l'étape V) est acheminée et à travers l'espace extérieur de tubes duquel un milieu chauffant est acheminé, ou à travers l'espace extérieur de tubes duquel la phase liquide restante après la détente dans l'étape V) est acheminée et à travers l'espace intérieur de tubes duquel un milieu chauffant est acheminé.

**11.** Procédé selon l'une quelconque des revendications précédentes, selon lequel les échangeurs de chaleur utilisés dans l'étape I) et dans l'étape II) sont choisis indépendamment l'un de l'autre dans le groupe constitué par les échangeurs de chaleur à faisceau de tubes et les échangeurs de chaleur à plaques.

**12.** Procédé selon l'une quelconque des revendications précédentes, selon lequel les échangeurs de chaleur utilisés dans l'étape I) et dans l'étape II) sont exploités indépendamment l'un de l'autre avec un milieu caloporteur choisi dans le groupe constitué par une huile, des masses fondues salines, des solvants organiques et de l'eau.

**13.** Procédé selon l'une quelconque des revendications précédentes, selon lequel une soupape de maintien de pression pour la phase gazeuse se formant et une régulation du niveau de liquide pour la phase liquide sont agencés dans la zone de séparation de l'étape IV), grâce auxquelles la pression de la phase gazeuse est maintenue constante.

**14.** Procédé selon l'une quelconque des revendications précédentes, selon lequel l'étape IV) comporte :

- l'entrée de la valeur de consigne pour la température du mélange réactionnel de l'étape III) dans une unité électronique de traitement de données ;
- la fourniture des valeurs mesurées de la température réelle du mélange réactionnel de l'étape III) sous forme électronique ;
- la transmission des valeurs mesurées fournies sous forme électronique à l'unité électronique de traitement de données ;
- la comparaison de la valeur de consigne à la température réelle dans l'unité électronique de traitement de données ;
- lorsqu'une déviation de la température réelle de la valeur de consigne de plus de 1,0 °C est établie :

- en cas de déviation vers le haut, la réduction
- en cas de déviation vers le bas, l'augmentation de la température du milieu caloporteur utilisé dans les échangeurs de chaleur par transmission d'une commande appropriée à partir de l'unité électronique de traitement de données au dispositif de conditionnement en température utilisé pour le conditionnement en température du milieu caloporteur.

**15.** Procédé selon l'une quelconque des revendications précédentes, selon lequel

(i) du diisocyanate de méthylène-diphénylène et/ou du polyisocyanate de polyméthylène-polyphénylène sont fabriqués par mise en réaction de méthylène-diphénylène-diamine et/ou de polyméthylène-polyphénylène-polyamine avec du phosgène, ou
(ii) du diisocyanate de toluylène est fabriqué par mise en réaction de toluylène-diamine avec du phosgène.

**EP 3 653 605 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3403204 A
- DE 1768439 A
- DE 10222968 A
- EP 1873142 A1
- EP 1616857 A1
- EP 1854783 A2
- EP 0830894 A1
- EP 2077150 A1